# EUROPEAN PATENT SPECIFICATION

(11) **EP 3 701 804 B1**
(45) Date of publication and mention of the grant of the patent: **08.05.2024**
(21) Application number: 18869790.8
(22) Date of filing: 14.09.2018
(51) Int. Cl.: A23L 2/52, A23L 2/00, A23L 2/70, A61K 9/08, A61K 31/4745, A61K 47/22, A61K 47/40, A61P 1/16, A61P 3/02, A61P 17/02, A61P 27/12, A61P 31/12, A61P 35/00, A61P 37/08, A61P 43/00, A23L 2/68

(54) **PYRROLOQUINOLINE QUINONE-CONTAINING ACIDIC BEVERAGE AND METHOD FOR SUPPRESSING DEPOSITION OF PYRROLOQUINOLINE QUINONE**
SAURES GETRÄNK ENTHALTEND PYRROLOCHINOLINCHINON UND VERFAHREN ZUR UNTERDRÜCKUNG DER AUSFÄLLUNG VON PYRROLOCHINOLINCHINON
BOISSON ACIDE CONTENANT DE LA PYRROLOQUINOLÉINE QUINONE ET PROCÉDÉ DE SUPPRESSION DE LA PRÉCIPITATION DE PYRROLOQUINOLÉINE QUINONE

(30) Priority: 25.10.2017 JP 2017206347
(43) Date of publication of application: 02.09.2020
(73) Proprietor: Mitsubishi Gas Chemical Company, Inc., Tokyo 100-8324 (JP)
(72) Inventor: SUGIMOTO, Atsushi, Niigata-shi Niigata 950-3112 (JP); IKEMOTO, Kazuto, Niigata-shi Niigata 950-3112 (JP)
(74) Representative: Müller-Boré & Partner Patentanwälte PartG mbB
(86) International application number: PCT/JP2018/034119
(87) International publication number: WO 2019/082548

(56) References cited:
- JP-A- 2011 505 166
- JP-A- 2012 180 319
- JP-A- 2012 180 319
- JP-A- 2014 214 089
- JP-A- 2016 518 143
- US-A1- 2009 191 320
- HIDAKA Toru et al.: "Eds. Food Additives Dictionary", Food Additive Encyclopedia 2001, 2001, pages 8-9, XP009520421, ISBN: 4-916143-07-8

## Description

### Technical Field

The present invention relates to an acidic beverage and a method for suppressing deposition.

### Background Art

Acidic beverages are highly preferred beverages that have pH in an acidic region and are characterized by modest sourness and refreshing flavor. Ascorbic acid is widely used for adjusting the pH or flavor of such acidic beverages and preventing oxidation.

Pyrroloquinoline quinone is a coenzyme present in bacteria as well as molds and yeasts which are eukaryotes. The possibility has been proposed that the pyrroloquinoline quinone is a novel vitamin. Thus, the pyrroloquinoline quinone has received attention as a substance useful for health supplements, cosmetics, and the like. Also, the pyrroloquinoline quinone has previously been found to have many physiological activities such as a cell growth promoting effect, an anti-cataract effect, a prophylactic or therapeutic effect on liver diseases, a wound healing effect, an antiallergic effect, a reverse transcriptase inhibitory effect and a glyoxalase I inhibitory effect (anticancer effect) (see, for example, Non Patent Literature 1). An inclusion body of pyrroloquinoline quinone with cyclodextrin has previously been reported in order to stabilize the pyrroloquinoline quinone (see, for example, Patent Literature 1).

### Citation List

### Patent Literature

Patent Literature 1: Japanese Patent Laid-Open No. 2012-180319

### Non Patent Literature

Non Patent Literature 1: Biosci Biotechnol Biochem. 2016; 80 (1): 13-22. doi:10.1080/09168451.2015.1062715. Further, JP 2012 180319 A describes a pyrroloquinoline quinone having improved stability and a production method thereof.

### Summary of Invention

### Technical Problem

The present inventors have conducted studies on pyrroloquinoline quinone-containing acidic beverages and consequently found that a problem of pyrroloquinoline quinone is that deposition occurs in the presence of ascorbic acid.

Accordingly, an object of the present invention is to provide a favorable acidic beverage that is less likely to deposit pyrroloquinoline quinone in the presence of ascorbic acid.

### Solution to Problem

As a result of conducting studies, the present inventors have found that the addition of cyclodextrin to an acidic beverage containing pyrroloquinoline quinone and ascorbic acid suppresses the deposition of the pyrroloquinoline quinone.

The present invention is set out in the appended set of claims.

### Advantageous Effects of Invention

The present invention can provide an acidic beverage that does not deposit pyrroloquinoline quinone even in the presence of ascorbic acid.

### Description of Embodiments

Hereinafter, the mode for carrying out the present invention (hereinafter, referred to as the "present embodiment") will be described in detail.

The acidic beverage according to the first embodiment of the present invention contains (A) pyrroloquinoline quinone and/or a salt thereof, (B) ascorbic acid, (C) cyclodextrin, and optionally (D) an acidulant other than ascorbic acid and an additional component. All the components including pyrroloquinoline quinone are dissolved in the presence of cyclodextrin in the acidic beverage.

The acidic beverage of the present embodiment contains pyrroloquinoline quinone (hereinafter, also referred to as "PQQ") and/or a salt thereof as a component (A).

In the present embodiment, PQQ is a compound represented by formula 1.

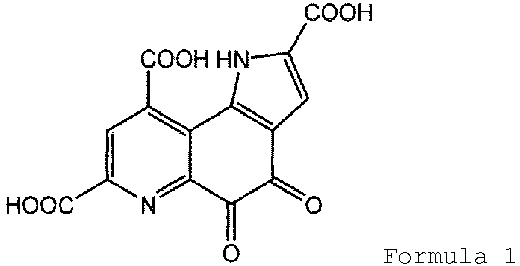

Examples of the salt of PQQ include, but are not particularly limited to, metal salts, for example, monosodium salt, disodium salt, trisodium salt, monopotassium salt, dipotassium salt, tripotassium salt, monolithium salt, dilithium salt, and trilithium salt. Disodium salt is preferred.

When the component (C) is α-cyclodextrin, the content of the component (A) in the acidic beverage of the present embodiment is 5 mg/L to 100 mg/L, preferably 10 mg/L to 100 mg/L, more preferably 10 mg/L to 40 mg/L. When the component (C) is γ-cyclodextrin, the content of the component (A) in the acidic beverage of the present embodiment is 5 mg/L to 550 mg/L, preferably 10 mg/L to 80 mg/L, more preferably 10 mg/L to 40 mg/L.

The content of the component (A) can be measured by an analysis method suitable for the situation of a measurement sample, among usually known methods for analyzing pyrroloquinoline quinone. Specifically, the component (A) can be quantitatively analyzed by liquid chromatography described in Examples mentioned later. A sample containing pyrroloquinoline quinone may be appropriately treated, if necessary. For example, the sample can be freeze-dried for adaptation to the detection range of an apparatus for measurement, or impurities in the sample can be removed for adaptation to the separating ability of an apparatus.

The ascorbic acid serving as the component (B) can be added as an acidulant, an antioxidant, or the like to the acidic beverage. The ascorbic acid is blended thereinto in the same amount or a larger amount than that of the pyrroloquinoline quinone serving as the component (A). Specifically, the mass ratio of the component (B) to the component (A) [(B)/(A)] is preferably 1 to 1000, more preferably 2 to 350, particularly preferably 10 to 100. When the ascorbic acid has a mass ratio of less than 1 to the pyrroloquinoline quinone, no deposition problem arises. On the other hand, if the ascorbic acid has a mass ratio of more than 1000 to the pyrroloquinoline quinone, the balance of taste is deteriorated.

The cyclodextrin used is any of α- and γ-cyclodextrins without limitations. γ-Cyclodextrin is preferred from the viewpoint of a deposition suppressing effect. When cyclodextrin is γ-cyclodextrin, the content of the γ-cyclodextrin in the acidic beverage is 0.3 to 24% by weight, preferably 0.4 to 13% by weight, more preferably 1 to 10% by weight.

When cyclodextrin is α-cyclodextrin, the content of the α-cyclodextrin in the acidic beverage is 3 to 13% by weight, preferably 5 to 13% by weight, more preferably 6 to 10% by weight.

Even if the concentration of the cyclodextrin falls outside the range described above, the cyclodextrin can decrease the occurrence of deposits as compared with the case of adding no cyclodextrin. However, if the concentration of the cyclodextrin falls below the lower limit, the deposition of pyrroloquinoline quinone may be observed. If the concentration of the cyclodextrin exceeds the upper limit, the cyclodextrin itself may be deposited.

Examples of the acidulant (D) include, but are not particularly limited to, citric acid, lactic acid, malic acid, phosphoric acid, and succinic acid. In the acidic beverage of the present embodiment, the acidulant is effective for producing a feeling of refreshment. The content of the acidulant can be appropriately set in terms of gustation, and is, for example, preferably 0.001 to 5% by weight, more preferably 0.01 to 3% by weight, particularly preferably 0.1 to 3% by weight.

The pH of the acidic beverage of the present embodiment is preferably 2 to 5.4, more preferably 2 to 4, particularly preferably 2 to 3. Acidity is necessary for producing a feeling of refreshment. The pH is controllable by blending an appropriate amount of the acidulant thereinto.

The acidic beverage of the present embodiment preferably contains a sweetener. Examples of the sweetener include carbohydrate-based sweeteners and high-intensity sweeteners. In this context, the "high-intensity sweetener" herein means an artificial or natural sweetener that has 10 times to 1000 times the sweetness of sucrose and can impart sweetness to a food or drink by addition in a small amount.

Examples of the carbohydrate-based sweetener include: monosaccharides such as fructose, glucose, tagatose, arabinose, D-psicose, and D-allose; disaccharides such as lactose, trehalose, maltose, sucrose, and cellobiose; and sugar alcohols such as erythritol, xylitol, maltitol, sorbitol, mannitol, maltitol, reduced palatinose, lactitol, and reduced starch saccharification products. Examples of the high-intensity sweetener include sucralose, acesulfame potassium, aspartame, stevia (rebaudioside and stevioside), thaumatin, saccharin, saccharin sodium, licorice extracts, *Siraitia grosvenorii* extracts, neotame, mabinlin, brazzein, monellin, glycyrrhizin, alitame, sodium cyclohexylsulfamate, dulcin, and neohesperidin. One or two or more of these sweeteners can be used. The high-intensity sweetener is preferably one or two or more members selected from sucralose, acesulfame potassium, aspartame, stevia and thaumatin.

The content of the sweetener is appropriately adjusted according to the degree of sweetness required for a final product. The content of the sweetener is, for example, preferably 1 to 20% by weight, more preferably 1 to 15% by weight, particularly preferably 1 to 10% by weight, in terms of sucrose.

The acidic beverage of the present embodiment can contain carbonic acid in order to impart a feeling of stimulation to its taste.

The content of the carbonic acid is preferably 0.01 to 1% by weight, more preferably 0.1 to 0.5% by weight, particularly preferably 0.3 to 0.4% by weight.

The acidic beverage of the present embodiment may contain an additive that is usually blended into beverages, without inhibiting the desired effect. Examples of such an additive include, but are not particularly limited to, colorants, antioxidants, flavors, salts, and stabilizers. Examples of the colorants can include, but are not particularly limited to, caramel colors, gardenia colors, fruit juice colors, vegetable colors, and synthetic pigments. Examples of the antioxidants can include, but are not particularly limited to, vitamin C, vitamin E, and polyphenol. Examples of the salts can include, but are not particularly limited to, sodium chloride and potassium chloride. Examples of the stabilizers can include, but are not particularly limited to, pectin and water-soluble soybean polysaccharides.

The acidic beverage of the present embodiment may also contain an additional additive according to the type of a final product. Examples of the additional additive include, but are not particularly limited to, eudesmol, inulin, procyanidin C1, nootkatone, methoxyflavone, hydroxytyrosol, theanine, l-menthol, vanillin, proanthocyanidin, saponin, rutin, capsaicin, fructan, strictinin, astaxanthin, placenta, anthocyanin, glucomannan, arabinoxylan, acrylamide, sphingomyelin, citrulline, furfuryl thiol, isomaltulose, collagen peptide, casein peptide, amino acids (proline, glycine, etc.), L-arabinose, 2,5-piperazinedione,3,6-bis(phenylmethyl)-,(3S,6S),6-O-PUFA ascorbic acid ester, α-glucan, β-glucan, chlorogenic acid, pyruvic acid, dicaffeoylquinic acid, dehydroascorbic acid, α acid or iso-α acid, β acid, orotic acid, lipoic acid, oligosaccharides (maltooligosaccharide, etc.), isoquercitrin or sugar adducts thereof, hesperidin and sugar adducts of hesperidin, octenyl succinate starch, coenzyme Q10, polymerized or non-polymerized catechins, chlorophylls, theaflavins, malt extracts, plant extracts, aroma extracts, caffeine, turmeric, fruit flavors, fruit juices, and mesophyll.

The acidic beverage of the present embodiment may further contain a functional component other than pyrroloquinoline quinone. Examples of the functional component include, but are not particularly limited to, BCAA (branched chain amino acids), amino acids (histidine, L-serine, essential amino acids containing 40% leucine, etc.), β-cryptoxanthin, DHA (docosahexaenoic acid), EPA (eicosapentaenoic acid), GABA (γ-aminobutyric acid), acetic acid, HMB (bis-3-hydroxy-3-methyl butyrate monohydrate), N-acetylglucosamine, astaxanthin, isoflavone (kudzu (Pueraria) flower-derived isoflavone, etc.), ginkgo leaf terpene lactone, ginkgo leaf flavonoid glycoside, epigallocatechin gallate, methylated catechin, ornithine, cacao flavanol, lactic acid bacteria (*Lactobacillus gasseri* SBT2055, etc.), bifidobacteria, agar-derived galactan, guar gum decomposition products, glucosamine, glucosylceramide, *Plantago psyllium* seed coat-derived dietary fiber, Salacia-derived salacinol, tiliroside, sodium hyaluronate, piperine, monoglucosyl hesperidin, lactotripeptide, imidazole dipeptide, lycopene, and apple polyphenol.

The acidic beverage of the present embodiment can be produced, for example, by dissolving the components (A) to (C) mentioned above in water.

The acidic beverage of the present embodiment may be heat-sterilized at the desired timing after preparation. The heat sterilization method is not particularly limited as long as the method is appropriate for conditions stipulated by a law or a regulation (in Japan, Food Sanitation Act) that should be applied thereto. Examples thereof can include a retort sterilization method, a high temperature-short time method (HTST), an ultrahigh temperature method (UHT), and pasteurization.

The heat sterilization method is appropriately selected. For example, retort sterilization or pasteurization can be adopted when a container such as a metal can or a bottle filled with the beverage can be directly heat-sterilized (e.g., 60 to 140°C, 1 to 60 min). For the pasteurization, the heat sterilization can be performed, for example, at 65°C for 1 to 60 minutes, preferably at 65°C for 5 to 30 minutes, more preferably at 65°C for 10 to 20 minutes.

As for a container, such as a PET bottle, which cannot be retort-sterilized, aseptic filling, hot-pack filling, or the like can be adopted which involves heat-sterilizing the beverage in advance under sterilization conditions (e.g., at 65 to 140°C for 0.1 seconds to 30 minutes, preferably at 70 to 125°C for 1 second to 25 minutes, more preferably at 75 to 120°C for 10 seconds to 20 minutes) equivalent to those described above, and filling a sterilized container with the resulting beverage in an aseptic environment.

According to the second embodiment, the present invention provides a method for suppressing the deposition of pyrroloquinoline quinone, comprising the step of bringing pyrroloquinoline quinone or a salt thereof into contact with cyclodextrin in the presence of ascorbic acid.

Each component for use in the method for suppressing deposition is as mentioned above. The amount of each component is appropriately determined according to the desired effect under the provision that the amount of cyclodextrin and pyrroloquinoline quinone or the salt thereof falls within the ranges as set out in the appended set of claims. The method for suppressing deposition may further comprise a step for use in the production of acidic beverages, for example, a step of the filling, sterilization, etc. mentioned above.

### Examples

The pyrroloquinoline quinone disodium salt used in the present embodiment was BioPQQ(Registered Trademark) manufactured by Mitsubishi Gas Chemical Co., Inc. α-Cyclodextrin and γ-cyclodextrin manufactured by Wako Pure Chemical Industries, Ltd. were used.

### [Example 1]

An aqueous solution containing 0.3% by weight of ascorbic acid, 0.6% by weight of citric acid, 4.0% by weight of α-cyclodextrin, and 0.008% by weight (80 mg/L) of pyrroloquinoline quinone disodium salt per L was prepared. The ascorbic acid/PQQ disodium salt mass ratio was 37.5. This aqueous solution was used as a pyrroloquinoline quinone-containing acidic beverage. The pH was 2.3. The pyrroloquinoline quinone-containing acidic beverage was preserved at 4°C for 1 day and evaluated for the presence or absence of the deposition of pyrroloquinoline quinone. The deposition of pyrroloquinoline quinone was not confirmed in the pyrroloquinoline quinone-containing acidic beverage blended with α-cyclodextrin.

### [Example 2]

A pyrroloquinoline quinone-containing acidic beverage containing 0.3% by weight of ascorbic acid, 0.6% by weight of citric acid, 4.0% by weight of γ-cyclodextrin, and 0.008% by weight (80 mg/L) of pyrroloquinoline quinone disodium salt per L was prepared. The ascorbic acid/PQQ disodium salt mass ratio was 37.5. It was 37.5. The pH was 2.3. This pyrroloquinoline quinone-containing acidic beverage was preserved at 4°C for 1 day and evaluated for the presence or absence of the deposition of pyrroloquinoline quinone. The deposition of pyrroloquinoline quinone was not confirmed in the acidic beverage blended with γ-cyclodextrin.

### [Comparative Example 1]

0.3% by weight of ascorbic acid, 0.6% by weight of citric acid, and 0.008% by weight (80 mg/L) of pyrroloquinoline quinone disodium salt were added per L in the same way as in Example 1 or 2 described above except that no cyclodextrin was added, to prepare a pyrroloquinoline quinone-containing acidic beverage. The ascorbic acid/PQQ disodium salt mass ratio was 37.5. The pH was 2.3. When the pyrroloquinoline quinone-containing acidic beverage was preserved at 4°C for 1 day, deposition occurred as dark red precipitation.

As is evident from these results, the addition of cyclodextrin is effective for suppressing the deposition of pyrroloquinoline quinone in an acidic solution.

### [Comparative Examples 2 to 4]

An acidic beverage containing 0.3% by weight of ascorbic acid, 0.6% by weight of citric acid, and 0.008% by weight (80 mg/L) of pyrroloquinoline quinone disodium salt per L was prepared in the same way as in Example 1 or 2 described above except that no cyclodextrin was added. The ascorbic acid/PQQ disodium salt mass ratio was 37.5.

Glucose, fructose, or sorbitol was added thereto at 5.0% by weight. The resulting acidic beverages were preserved at 4°C for 1 day to confirm the presence or absence of deposition. The results are shown in Table 1.

**[Table 1]**

| Comparative Example | Additive | Presence or absence of deposition |
|---|---|---|
| 2 | Glucose | Present |
| 3 | Fructose | Present |
| 4 | Sorbitol | Present |

The deposition suppressing effect is an effect brought about by cyclodextrin and was unable to be achieved by other sugars.

### [Reference Example 3]

The same experiment as in Example 1 was carried out such that the concentration of α-cyclodextrin was 2.0% by weight. The ascorbic acid/PQQ disodium salt mass ratio was 37.5.

As a result of preserving the acidic beverage at 4°C for 1 day, deposition occurred as dark red precipitation, the amount of which was however smaller than that of Comparative Example 1.

### [Examples 4 and 5]

The same experiment as in Example 1 was carried out such that the concentration of γ-cyclodextrin was 1.0 and 2.0% by weight. The ascorbic acid/PQQ disodium salt mass ratio was 37.5.

As a result of preserving the acidic beverages at 4°C for 1 day, deposition was confirmed in neither of the acidic beverages. The results are shown in Table 2.

**[Table 2]**

| Example | Additive | Additive concentration (% by weight) | Presence or absence of deposition |
|---|---|---|---|
| 4 | γ-Cyclodextrin | 1.0 | Absent |
| 5 | γ-Cyclodextrin | 2.0 | Absent |

### [Reference Example 6]

The same experiment as in Example 1 was carried out such that the concentration of γ-cyclodextrin was 0.2% by weight. As a result of preserving the acidic beverage at 4°C for 1 day, deposition occurred as dark red precipitation, the amount of which was however smaller than that of Comparative Example 1.

### [Examples 7 and 8]

An acidic aqueous solution containing 0.3% by weight of ascorbic acid, 0.6% by weight of citric acid, and 4.0% by weight of α-cyclodextrin per L was prepared in the same way as in Example 1 described above except that the amount of pyrroloquinoline quinone disodium salt added was changed. To this acidic aqueous solution, pyrroloquinoline quinone disodium salt was added at 0.001% by weight (Example 7) (10 mg/L) or 0.004% by weight (Example 8) (40 mg/L) to prepare acidic beverages. After preservation of the acidic beverages at 4°C for 1 day, precipitation was confirmed in neither of the acidic beverages. The results are shown in Table 3 below. The ascorbic acid/PQQ disodium salt mass ratio in Example 7 was 300. The ascorbic acid/PQQ disodium salt mass ratio in Example 8 was 75.

**[Table 3]**

| Example | Additive | PQQ disodium salt concentration | Presence or absence of deposition |
|---|---|---|---|
| 7 | α-Cyclodextrin | 10 | Absent |
| 8 | α-Cyclodextrin | 40 | Absent |

### [Comparative Example 5]

The same experiment as in Examples 7 and 8 was carried out except that the concentration of pyrroloquinoline quinone disodium salt was 0.02% by weight per L. As a result of preserving the acidic beverage at 4°C for 1 day, deposition occurred as dark red precipitation. The ascorbic acid/PQQ disodium salt mass ratio was 15.

### [Examples 9 to 11]

An acidic aqueous solution containing 0.3% by weight of ascorbic acid, 0.6% by weight of citric acid, and 2.0% by weight of γ-cyclodextrin was prepared in the same way as in Example 5 described above except that the amount of pyrroloquinoline quinone disodium salt added was changed. To this acidic aqueous solution, pyrroloquinoline quinone disodium salt was added at a concentration per L of 0.001% by weight (Example 9) (10 mg/L), 0.004% by weight (Example 10) (40 mg/L), or 0.02% by weight (Example 11) (200 mg/L) to prepare acidic beverages. After preservation of the acidic beverages at 4°C for 1 day, precipitation was confirmed in neither of the acidic beverages. The results are shown in Table 4 below.

**[Table 4]**

| Example | Additive | PQQ disodium salt concentration (mg/L) | Presence or absence of deposition | Ascorbic acid/PQQ disodium salt |
|---|---|---|---|---|
| 9 | γ-Cyclodextrin | 10 | Absent | 300 |
| 10 | γ-Cyclodextrin | 40 | Absent | 75 |
| 11 | γ-Cyclodextrin | 200 | Absent | 15 |

The present invention can provide a stable beverage in which the deposition of pyrroloquinoline quinone is suppressed even in the presence of ascorbic acid.

### [Example 12]

The components given below were added to Wilkinson's carbonated water bottle to prepare a carbonated beverage. 0.5% by weight of table sugar, 0.05% by weight of citric acid, 0.3% by weight of ascorbic acid, 0.4% by weight of γ-cyclodextrin, 0.008% by weight (80 mg/L) of pyrroloquinoline quinone disodium salt, and 0.015% by weight of acesulfame K were added per L. The ascorbic acid/PQQ disodium salt mass ratio was 37.5. The pH was 3. While this beverage was preserved at 4°C, no deposition was confirmed. The beverage was sweet with a feeling of refreshment.

### [Comparative Example 6]

The components given below were added to Wilkinson's carbonated water bottle to prepare a carbonated beverage. 0.5% by weight of table sugar, 0.05% by weight of citric acid, 0.3% by weight of ascorbic acid, 0.006% by weight (60 mg/L) of pyrroloquinoline quinone disodium salt, and 0.015% by weight of acesulfame K were added per L. The ascorbic acid/PQQ disodium salt mass ratio was 50. The pH was 3. While this beverage was preserved at 4°C, pyrroloquinoline quinone was deposited. The beverage was sweet with a feeling of refreshment.

According to the present embodiment, deposition was also able to be suppressed for the carbonated beverage. Particularly, γ-cyclodextrin was found to have high ability to suppress deposition and prevent deposition of pyrroloquinoline quinone even having a higher concentration. The addition of cyclodextrin was also found to have no influence on taste and be excellent for beverage production.

### [Examples 13, 14, 17, 18, and 20 to 24; Reference Examples 15, 16, and 19]

Ascorbic acid, citric acid, α-cyclodextrin (α-CD) or γ-cyclodextrin (γ-CD), and pyrroloquinoline quinone disodium salt (PQQ disodium salt) were added as shown in Tables 5 and 6 to prepare aqueous solutions. These aqueous solutions were used as pyrroloquinoline quinone-containing acidic beverages. The pH of each acidic beverage was 2. The acidic beverages were preserved at 4°C for 1 day and evaluated for the deposition of the acidic beverage components. The evaluation results are shown in Tables 5 and 6.

**[Table 5]**

| Example | α-CD | PQQ disodium salt | Ascorbic acid (ascorbic acid/PQQ disodium salt) | Citric acid | Deposition |
|---|---|---|---|---|---|
| 13 | 10% | 0.008% (80 mg/L) | 0.3% (37.5) | 0.6% | Absent |
| 14 | 12% | 0.008% (80 mg/L) | 0.3% (37.5) | 0.6% | Absent |
| 15* | 14% | 0.008% (80 mg/L) | 0.3% (37.5) | 0.6% | Present |
| 16* | 15% | 0.008% (80 mg/L) | 0.3% (37.5) | 0.6% | Present |

| | | | | | |
|---|---|---|---|---|---|
| * Reference Example | | | | | |

**[Table 6]**

| Example | γ-CD | PQQ disodium salt | Ascorbic acid (ascorbic acid/PQQ disodium salt) | Citric acid | Deposition |
|---|---|---|---|---|---|
| 17 | 20% | 0.008% (80 mg/L) | 0.3% (37.5) | 0.6% | Absent |
| 18 | 23% | 0.008% | 0.3% (37.5) | 0.6% | Absent |
| 19* | 25% | 0.008% (80 mg/L) | 0.3% (37.5) | 0.6% | Present |
| 20 | 4% | 0.024% (240 mg/L) | 0.3% (12.5) | 0.6% | Absent |
| 21 | 4% | 0.028% (280 mg/L) | 0.3% (10.7) | 0.6% | Absent |
| 22 | 4% | 0.032% (320 mg/L) | 0.3% (9.4) | 0.6% | Absent |
| 23 | 4% | 0.036% (360 mg/L) | 0.3% (8.3) | 0.6% | Absent |
| 24 | 4% | 0.04% (400 mg/L) | 0.3% (7.5) | 0.6% | Absent |

| | | | | | |
|---|---|---|---|---|---|
| *Reference Example | | | | | |

The addition of cyclodextrin at any of the concentrations decreased deposits as compared with the case of adding no cyclodextrin. Particularly, α-cyclodextrin having a concentration of less than 14% completely suppressed deposition. γ-Cyclodextrin having a concentration of less than 25% completely suppressed deposition.

## Claims

1. An acidic beverage comprising:
(A) pyrroloquinoline quinone or a salt thereof;
(B) ascorbic acid; and
(C) cyclodextrin,
wherein when the component (C) is α-cyclodextrin, the content of the component (A) is 5 mg/L to 100 mg/L in the acidic beverage, and the α-cyclodextrin in the acidic beverage is 3 to 13% by weight;
wherein when the component (C) is γ-cyclodextrin, the content of the component (A) is preferably 5 mg/L to 550 mg/L in the acidic beverage, and the γ-cyclodextrin in the acidic beverage is 0.3 to 24% by weight.

2. The acidic beverage according to claim 1, wherein the component (C) is α-cyclodextrin, and a content thereof is 3 to 13% by weight.

3. The acidic beverage according to claim 1, wherein the component (C) is γ-cyclodextrin, and a content thereof is 0.3 to 24% by weight.

4. The acidic beverage according to any one of claims 1 to 3, wherein a mass ratio of the component (B) to the component (A) [(B)/(A)] is 1 to 1000.

5. The acidic beverage according to any one of claims 1 to 4, wherein the acidic beverage has pH of 2 to 5.4.

6. The acidic beverage according to any one of claims 1 to 5, further comprising an acidulant other than ascorbic acid.

7. The acidic beverage according to any one of claims 1 to 6, further comprising a sweetener.

8. The acidic beverage according to any one of claims 1 to 7, further comprising carbonic acid.

9. A method for suppressing the deposition of pyrroloquinoline quinone in an acidic beverage, comprising the step of bringing pyrroloquinoline quinone or a salt thereof into contact with cyclodextrin in the presence of ascorbic acid,
wherein when the cyclodextrin is α-cyclodextrin, the content of the pyrroloquinoline quinone or the salt thereof is 5 mg/L to 100 mg/L in the acidic beverage, and the α-cyclodextrin in the acidic beverage is 3 to 13% by weight;
wherein when the cyclodextrin is γ-cyclodextrin, the content of the pyrroloquinoline quinone or the salt thereof is preferably 5 mg/L to 550 mg/L in the acidic beverage, and the γ-cyclodextrin in the acidic beverage is 0.3 to 24% by weight.

## Patentansprüche

1. Saures Getränk, umfassend:
(A) Pyrrolochinolinchinon oder ein Salz davon;
(B) Ascorbinsäure; und
(C) Cyclodextrin,
wobei, wenn die Komponente (C) α-Cyclodextrin ist, der Gehalt der Komponente (A) in dem sauren Getränk 5 mg/L bis 100 mg/L beträgt und das α-Cyclodextrin in dem sauren Getränk 3 bis 13 Gew.-% beträgt;
wobei, wenn die Komponente (C) γ-Cyclodextrin ist, der Gehalt der Komponente (A) in dem sauren Getränk vorzugsweise 5 mg/L bis 550 mg/L beträgt und das γ-Cyclodextrin in dem sauren Getränk 0,3 bis 24 Gew.-% beträgt.

2. Saures Getränk nach Anspruch 1, wobei die Komponente (C) α-Cyclodextrin ist und ein Gehalt davon 3 bis 13 Gew.-% beträgt.

3. Saures Getränk nach Anspruch 1, wobei die Komponente (C) γ-Cyclodextrin ist und ein Gehalt davon 0,3 bis 24 Gew.-% beträgt.

4. Saures Getränk nach einem der Ansprüche 1 bis 3, wobei ein Massenverhältnis der Komponente (B) zu der Komponente (A) [(B)/(A)] 1 zu 1000 beträgt.

5. Saures Getränk nach einem der Ansprüche 1 bis 4, wobei das saure Getränk einen pH-Wert von 2 bis 5,4 aufweist.

6. Saures Getränk nach einem der Ansprüche 1 bis 5, weiter umfassend ein anderes Säuerungsmittel als Ascorbinsäure.

7. Saures Getränk nach einem der Ansprüche 1 bis 6, weiter umfassend einen Süßstoff.

8. Saures Getränk nach einem der Ansprüche 1 bis 7, weiter umfassend Kohlensäure.

9. Verfahren zur Unterdrückung der Ablagerung von Pyrrolochinolinchinon in einem sauren Getränk, umfassend den Schritt, Pyrrolochinolinchinon oder ein Salz davon in Gegenwart von Ascorbinsäure mit Cyclodextrin in Kontakt zu bringen,
wobei, wenn das Cyclodextrin α-Cyclodextrin ist, der Gehalt des Pyrrolochinolinchinons oder des Salzes davon 5 mg/L bis 100 mg/L in dem sauren Getränk beträgt und das α-Cyclodextrin in dem sauren Getränk 3 bis 13 Gew.-% beträgt;
wobei, wenn das Cyclodextrin γ-Cyclodextrin ist, der Gehalt des Pyrrolochinolinchinons oder des Salz davon vorzugsweise 5 mg/L bis 550 mg/L in dem sauren Getränk beträgt und das γ-Cyclodextrin in dem sauren Getränk 0,3 bis 24 Gew.-% beträgt.

## Revendications

1. Boisson acide comprenant :
(A) de la pyrroloquinoléine quinone ou un sel de celle-ci ;
(B) de l'acide ascorbique ; et
(C) de la cyclodextrine,
dans laquelle lorsque le composant (C) est l'α-cyclodextrine, la teneur en le composant (A) est de 5 mg/l à 100 mg/l dans la boisson acide, et l'α-cyclodextrine dans la boisson acide est de 3 à 13 % en poids ;
dans laquelle lorsque le composant (C) est la y-cyclodextrine, la teneur en le composant (A) est de préférence de 5 mg/l à 550 mg/l dans la boisson acide, et la γ-cyclodextrine dans la boisson acide est de 0,3 à 24 % en poids.

2. Boisson acide selon la revendication 1, dans laquelle le composant (C) est l'α-cyclodextrine, et une teneur en celle-ci est de 3 à 13 % en poids.

3. Boisson acide selon la revendication 1, dans laquelle le composant (C) est la γ-cyclodextrine, et une teneur en celle-ci est de 0,3 à 24 % en poids.

4. Boisson acide selon l'une quelconque des revendications 1 à 3, dans laquelle un rapport massique du composant (B) sur le composant (A) [(B)/(A)] est de 1 sur 1000.

5. Boisson acide selon l'une quelconque des revendications 1 à 4, dans laquelle la boisson acide a un pH de 2 à 5,4.

6. Boisson acide selon l'une quelconque des revendications 1 à 5, comprenant en outre un acidulant autre que l'acide ascorbique.

7. Boisson acide selon l'une quelconque des revendications 1 à 6, comprenant en outre un édulcorant.

8. Boisson acide selon l'une quelconque des revendications 1 à 7, comprenant en outre de l'acide carbonique.

9. Procédé de suppression du dépôt de pyrroloquinoléine quinone dans une boisson acide, comprenant l'étape consistant à amener de la pyrroloquinoléine quinone ou un sel de celle-ci en contact avec de la cyclodextrine en présence d'acide ascorbique,
dans lequel lorsque la cyclodextrine est l'α-cyclodextrine, la teneur en la pyrroloquinoléine quinone ou le sel de celle-ci est de 5 mg/l à 100 mg/l dans la boisson acide, et l'α-cyclodextrine dans la boisson acide est de 3 à 13 % en poids ;
dans lequel lorsque la cyclodextrine est la γ-cyclodextrine, la teneur en la pyrroloquinoléine quinone ou le sel de celle-ci est de préférence de 5 mg/l à 550 mg/l dans la boisson acide, et la γ-cyclodextrine dans la boisson acide est de 0,3 à 24 % en poids.
